# EUROPEAN PATENT SPECIFICATION

(11) **EP 4 262 624 B1**
(45) Date of publication and mention of the grant of the patent: **07.05.2025**
(21) Application number: 21830481.4
(22) Date of filing: 15.12.2021
(51) Int. Cl.: A61F 2/06, A61B 34/10, A61F 2/04, A61B 6/00, A61F 2/24, A61B 6/50

(54) **MODEL AND METHOD OF MODELLING A PULMONARY AUTOGRAFT**
MODELL UND VERFAHREN ZUR MODELLIERUNG EINES PULMONALEN AUTOTRANSPLANTATS
MODÈLE ET PROCÉDÉ DE MODÉLISATION D'UNE AUTOGREFFE PULMONAIRE

(30) Priority: 16.12.2020 GB 202019871
(43) Date of publication of application: 25.10.2023
(73) Proprietor: Exstent Limited, Gloucestershire GL20 8DN (GB)
(72) Inventor: THORNTON, Warren Elliot, Bedfordshire MK45 5BE (GB); AUSTIN, Brian McConnell, London SE26 6HH (GB); GOLESWORTHY, Taliesin John, Cheltenham Gloucestershire GL53 7RW (GB)
(74) Representative: Barker Brettell LLP
(86) International application number: PCT/GB2021/053299
(87) International publication number: WO 2022/129897

(56) References cited:
- US-A1- 2009 292 349
- US-A1- 2018 245 243
- HELEN DORMAND ET AL: "Cardiovascular Magnetic Resonance in Marfan syndrome", JOURNAL OF CARDIOVASCULAR MAGNETIC RESONANCE, BIOMED CENTRAL LTD, LONDON UK, vol. 15, no. 1, 15 April 2013 (2013-04-15), pages 33, XP021148413, ISSN: 1532-429X, DOI: 10.1186/1532-429X-15-33
- CARREL THIERRY ET AL: "Long-Term Clinical and Imaging Follow-Up After Reinforced Pulmonary Autograft Ross Procedure", SURGERY ANNUAL, ELSEVIER, AMSTERDAM, NL, vol. 19, no. 1, 6 April 2016 (2016-04-06), pages 59 - 62, XP029494852, ISSN: 1092-9126, DOI: 10.1053/J.PCSU.2015.11.005

## Description

This invention relates to a support for a pulmonary autograft and methods of modelling a prospective pulmonary autograft and manufacturing a support for a pulmonary autograft.

The Ross procedure was first described by Donald Ross in 1967. In the Ross procedure, the healthy pulmonary valve and a part of the pulmonary trunk is used as an autograft to replace the diseased aortic valve. The Ross procedure can provide long-term viability of the aortic valve substitute, which translates into improved outcomes both in terms of patient survival and quality of life.

In its initial description, the operation was performed using a sub-coronary technique. In the sub-coronary technique, the pulmonary valve including all three sinuses and leaflets, plus some of the pulmonary artery is implanted in a sub-coronary position inside of the left ventricular outflow tract. However because it is a very technically demanding procedure, it did not gain wide acceptance amongst cardiac surgeons.

Although results of this technique were encouraging, the Ross procedure did not gain widespread popularity until the late 1980s when surgeons started to implant the pulmonary autograft as a freestanding root replacement (freestanding root replacement Ross operation) with reimplantation of the coronary arteries into the autograft. This technique is technically easier to perform than the sub-coronary implantation; however, frequent dilatation of the pulmonary autograft was observed using the freestanding root replacement Ross technique. Dilatation of the pulmonary autograft can result in valve insufficiency and the need for re-operation.

As such, it would be desirable in particular to reduce the need for re-operation (due to pulmonary autograft dilatation) if it could be avoided.

Supports adapted for location exteriorly of a blood vessel are known for use have been developed to counter aortic aneurysms, as disclosed in "Conservative Operating in the Management of Annular Dilation and Ascending Aortic Aneurysm" (Francis Robicsek and Mano Thubrikar, Ann Thorac Surg 1994;57:1672-4) which discusses a simple wrapping of the aorta in order to prevent further dilation. Our earlier application, WO 2004/026178 discloses supports which are manufactured such that they are shaped to be in morphological relationship with a patient's blood vessel in accordance with a computer-aided design (CAD) model of the blood vessel to counter dilation of the aorta in Marfan's Syndrome. For example, when modelling a patient's aorta it has been necessary to collect a set of adjacent 2-dimensional image slices of the aorta using a scanner and then reconstruct a 3-dimensional CAD model by stacking the 2-dimensional slices one on top of the other. A disadvantage of this method is that it is difficult to obtain suitable successive 2-dimensional images due to patient movement, e.g. breathing, within the scanner. Also, patient fatigue can be a problem due to the time that it takes to collect an appropriate number of images. In addition, MRI scanners are enclosed spaces and can induce claustrophobia in the patient.

Our further earlier application, WO2011/012894 (also published as US2009/292349) discloses a support for the ascending aorta again for use in combating aortic dilation associated with Marfan's syndrome, and a method of morphometric analysis of a patient's blood vessel using an imaging scanner comprising: (i) obtaining a diametral cross-sectional image of the blood vessel; (ii) obtaining a pseudo-transverse cross-section image of the blood vessel; and (iii) processing the images from steps (i) and (ii) to construct a morphometric model of the blood vessel.

We are also aware of Dormand, H., Mohiaddin, R.H. Cardiovascular Magnetic Resonance in Marfan syndrome J Cardiovasc Magn Reson 15, 33 (2013), which provides an overview of Marfan syndrome with an emphasis on cardiovascular complications and cardiovascular imaging. Both pre- and post-operative imaging is addressed with an explanation of surgical management. All relevant imaging modalities are discussed with a particular focus on cardiovascular magnetic resonance imaging.

In the context of the present invention a pulmonary autograft is a pulmonary artery and pulmonary valve in the aortic position, namely a person's own pulmonary artery and valve replacing the ascending aorta and aortic valve.

According to a first aspect of the invention as defined by the appended claims, there is provided a method of modelling an external support for a prospective pulmonary autograft attached to a patient's heart using an imaging scanner, the method comprising:
(i) obtaining, using the scanner, at least one scan of the vasculature of the heart during systole of the heart;
(ii) processing each scan using a processor to construct a model of the prospective pulmonary autograft; and
(iii) processing the model of the prospective pulmonary autograft to generate a model of the external support.

As such, we have appreciated that scanning during systole provides a more useful scan of the pulmonary valve, as it will be at the highest blood pressure applied by the right ventricle; the pulmonary valve will be at its largest diameter at that point in the cardiac cycle. Because the pulmonary autograft will be facing higher pressure in the aortic valve position, modelling the pulmonary valve at its maximum normal blood pressure allows the eventual support to support the pulmonary valve at a diameter that it can safely be stretched to.

In one embodiment obtaining, using the scanner, at least one scan of the heart vasculature during systole of the heart may comprise obtaining at least one scan including the pulmonary valve (PV), the pulmonary trunk (PT), the aortic root and ascending aorta, the aortic valve (AV), the left ventricular outflow tract (LVOT) and the right ventricular outflow tract (RVOT).

In one embodiment the method of the present invention, comprises obtaining, using the scanner, at least one scan of the heart vasculature during systole of the heart, wherein the at least one scan of the heart vasculature obtained during systole of the heart includes a scan of the pulmonary valve (PV), at least one scan of the pulmonary trunk (PT), at least one scan of the aortic root proximal to the Brachiocephalic branch, at least one scan of the left ventricular outflow tract (LVOT), and at least one scan of the right ventricular outflow tract (RVOT).

When a pulmonary valve for use in the Ross procedure is being scanned, the valve is not dilated nor regurgitant. This fact, and the very much lower blood pressures (BP) existing in the pulmonary vasculature/circulation, make identifying the pulmonary sinuses on a CT scan very difficult (when compared to higher pressure, diseased/dilated aortas used in the method of WO2011/012894).

In addition, compared to the left ventricular outflow track (LVOT) used in the method of WO2011/012894, the cross-sectional plan of the right ventricular outflow tract (RVOT) is a much poorer approximation to a circle which causes problems if the CAD modelling of WO2011/012894 is used. This makes the method disclosed in WO2011/012894 unsuitable for modelling a scanned RVOT.

Thus, we have appreciated that, if implants made with the method disclosed in WO2011/012894 are used to support a pulmonary autograft, these are found to be undersized causing surgical problems with their implantation and are likely to be of an anatomically inappropriate shape; for when relieved of pressure against the adjacent aortic structure, the pulmonary autograft assumes a more symmetrical tri-lobal planform than when it is in the pulmonary valve position.

Therefore the method disclosed in WO2011/012894 is unsuitable for a pulmonary autograft.

In one embodiment of the above method of modelling, the step of obtaining, using the scanner, at least one scan of the heart vasculature during systole of the heart comprises obtaining, using the scanner, at least one scan of the pulmonary valve during systole of the heart.

In one embodiment of the above method of modelling, the step of obtaining, using the scanner, at least one scan of the heart vasculature during systole of the heart comprises obtaining, using the scanner, at least one scan of the left ventricular outflow tract (LVOT) during systole of the heart.

In one embodiment of the above method of modelling, the step of obtaining, using the scanner, at least one scan of the heart vasculature during systole of the heart comprises obtaining, using the scanner, at least one scan of the pulmonary trunk (PT) up to the branch to each lung, during systole of the heart.

In one embodiment of the above method of modelling, the step of obtaining, using the scanner, at least one scan of the heart vasculature during systole of the heart comprises obtaining, using the scanner, at least one scan of the pulmonary valve, at least one scan of the aortic valve of the left ventricular outflow track (LVOT) and at least one scan of the pulmonary trunk.

A scanner suitable for use in the method according to the invention may be a MRI**,** MRA, X-ray CT, or a 3D pulsed Doppler Echo imaging scanner. Preferably the scanner used in the method of the present invention is a CT scanner.

In one embodiment, the method of modelling of the present invention may comprise obtaining, using a scanner, at least one scan of the heart vasculature during systole of the heart, wherein the at least one scan comprises obtaining at least one image selected from a diametrical cross section image and a pseudo-transverse cross-section image, of the heart vasculature as described above during systole of the heart. However, in a preferred embodiment, the method may comprise generating at least one aligned image in a plane aligned with the vasculature and in particular the prospective pulmonary autograft. Typically, each aligned image may be aligned with the pulmonary valve or the pulmonary trunk. There may be a trans-axial aligned image parallel to a plane of the pulmonary valve or perpendicular to the pulmonary trunk, and/or a sagittal aligned image perpendicular to the plane of the pulmonary valve or parallel to the pulmonary trunk.

In one embodiment the step of obtaining at least one scan of the heart vasculature during systole of the heart may comprise obtaining at least one scan of the heart vasculature in a temporal window during systole in the cardiac cycle of the patient's heart. Typically, the temporal window may be from a first point in the cardiac cycle to a second point in the cardiac cycle. The first point in the cardiac cycle may be 5%, 10% or 15% R-R (R-R being a commonly used reference to time through a cardiac cycle, being the percentage of the time from one R peak on an electrocardiogram to the next). The second point in the cardiac cycle may be between 20%, 25% or 30% R-R. In a preferred embodiment, the temporal window is between 10% R-R and 25% R-R in systole. The systolic scanning in this temporal window gives better approximation to the pulmonary valve at systemic blood pressures. Scanning in diastole, the sinuses are virtually non-existent (there is virtually a circular valve section), but in systole (particularly 10% to 25% R-R) the sinuses becomes visibly pronounced as 3 distinct lobes forming the wall of the valve. This allows safer identification of the pulmonary sinuses and also provides a better approximation to the size of the pulmonary valve when in the free-standing aortic position.

In one embodiment of the method of the present invention, the step of processing each scan using a processor to construct a model of the prospective pulmonary autograft may further comprise measuring one or more parameters from the at least one scan obtained of the heart vasculature during systole of the heart.

In one embodiment, the method of the present invention may comprise obtaining, using the scanner, at least one scan of the pulmonary valve during systole of the heart, at least one scan of the pulmonary trunk up to the branch to each lung during systole of the heart and at least one scan of the of the left ventricular outflow tract during systole of the heart, and measuring one or more parameters from the at least one scan of the pulmonary trunk and measuring one or more parameters from the at least one scan of the left ventricular outflow tract.

In one embodiment, the method of the present invention may comprise:
(i) obtaining, using the scanner, at least one scan of the heart vasculature during systole of the heart in the temporal window, wherein obtaining at least one scan of the heart vasculature comprises obtaining at least one scan of the pulmonary valve, obtaining at least one scan of the pulmonary trunk and obtaining at least one scan of the left ventricular outflow tract, during systole of the heart in the temporal window;
   and
(ii) processing each scan using a processor to construct a model of the prospective pulmonary autograft, wherein the processing further comprises measuring one or more parameters from the at least one scan of the pulmonary valve, measuring one or more parameters from the at least one scan of the pulmonary trunk and measuring one or more parameters from the at least one scan of the left ventricular outflow tract.

In one embodiment the method of the present invention further comprises measuring one or more parameters from the at least one scan of the heart vasculature during systole of the heart and using the one or more parameters to construct a model of the prospective pulmonary autograft.

The method according to the present invention may use at least four parameters measured from the at least one scan obtained of the heart vasculature during systole of the heart, for example in the temporal window, which are used to construct a model of the prospective pulmonary autograft. Preferably between 4 and 35 parameters are used to construct a model of the external support for the prospective pulmonary autograft, more preferably between 5 and 30 parameters, such as between 5 and 25 parameters, or between 6 and 30 parameters, or between 8 and 25 parameters or between 10 and 25 parameters.

In one embodiment at least four parameters are measured from the at least one scan of the heart vasculature to determine a model of the prospective pulmonary autograft. As described above the at least one scan of the heart vasculature may comprise at least one scan of the pulmonary valve, at least one scan of the pulmonary trunk and at least one scan of the left ventricular outflow tract.

Suitable parameters obtained from the above referenced scans may include a linear dimension, a circumferential dimension, a diametrical dimension and/or a rotational angle.

In the context of the present invention the term diameter refers to any polygonal approximations to a circle or an equivalent diameter, i.e. the trilobal cross-section of the Sinuses of Valsalva or the essentially circular cross section of the ascending pulmonary artery.

The proximal end of the pulmonary aorta has to be anastomosed onto the LVOT (i.e. the proximal end of the pulmonary autograft will be attached typically via stitching to the top of the left ventricular outflow tract (LVOT)). Thus, a suitable parameter for the method of the present invention may be the base diameter of the LVOT measured at systole (as discussed above). The base diameter may be measured at the aorto-ventricular junction - the anatomical junction between the distal end of the LVOT (furthest from the ventricle) and the proximal end of the aortic root. The base diameter of the LVOT may be measured from at least one scan of the LVOT during systole of the heart (e.g. between 10% R-R and 25% R-R). For example, the at least one scan of the LVOT during systole of the heart may comprise at least one transaxially aligned image of the LVOT in systole. This ensures that the prospective pulmonary autograft will fit where it has to attach/be anastomosed to the LVOT. In cases where the LVOT is less than 28mm diameter, the LVOT may be opened-up to 28mm diameter (e.g., Konno procedure) by the surgeon.

In one embodiment, further suitable parameters for the method of the present invention may comprise at least one of the diameters of each lobe of the sinuses of the prospective pulmonary autograft, the relative rotational spacings of the lobes and the distance of the circular centre of the lobes from the centreline of the lumen of the prospective pulmonary autograft. These parameters of the pulmonary valve may be measured from at least one scan of the pulmonary valve during systole of the heart (e.g. between 10% R-R and 25% R-R). These parameters are aimed to determine the shape of the cross section of the trilobal plane of the pulmonary valve.

In one embodiment, a further suitable parameter may be the perimeter of the pulmonary valve.

In one embodiment, a further suitable parameter may be the diameter of the pulmonary sinotubular junction (STJ) diameter. The pulmonary sinotubular junction (STJ) diameter may be measured from at least one scan of the pulmonary valve during systole of the heart (e.g. between 10% R-R and 25% R-R). The STJ is located at the top of the pulmonary valve, where the trilobal section (the three sinuses of the valve) transitions to a tubular shape (the pulmonary trunk).

In one embodiment, a further suitable parameter measured from the above referenced scan of the systolic heart vasculature may be the aortic diameter. The aortic diameter may be measured distal from the aortic valve and from at least one scan of the aortic artery, typically at around where the prospective pulmonary autograft is to be anastomosed to the ascending aorta. The scan may be during systole of the heart (e.g. between 10% R-R and 25% R-R). In one embodiment the distal "aortic" diameter is fixed at 30 or 32 mm when the measurement of the ascending aorta is 32 mm or more in diameter.

A further parameter may comprise a height of the pulmonary valve from a plane of the pulmonary ventricular junction (PVJ) to a plane of the pulmonary sinotubular junction.

One or more of the above described parameters allow the construction of a model of a prospective pulmonary autograft. This model is particularly useful in the freestanding Ross operation. In one embodiment the model may be a three-dimensional computer-generated model of the prospective pulmonary autograft.

In one embodiment, in the method of the present invention described herein, the processing step (ii) may further comprise a processor running computer aided design (CAD) software to construct a model of the prospective pulmonary autograft.

In order to generate the model of the external support, the method may take the model of the prospective pulmonary autograft and apply at least one transformation.

In one embodiment, the at least one transformation may comprise a shape transformation comprising equalising the diameters of the lobes. Typically, the two smaller lobe diameters may be increased to that of the largest lobe, although alternatively all three lobes could be adjusted to the average (typically mean or median) lobe diameter. This allows a better fit of the support on the prospective pulmonary autograft, bearing in mind that the prospective pulmonary autograft once transplanted will no longer have the adjacent aorta pressing against it, and will be facing increased blood pressure compared to when it is in its original location.

As such, the shape transformation may act to translate the centres of the lobes from forming an isosceles triangle to an equilateral triangle. The shape transformation may preserve the perimeter of the pulmonary valve.

The at least one transformation may comprise a scaling transformation. Whilst typically aortic CAD models have previously been scaled on diameter only to 100%, 95% or 90% or smaller or the size of the measured vessel depending on the degree of dilation and how much reduction in diameter the surgeon considers appropriate, in the present method the scaling transformation may comprise increasing the size of the model. Typically, the scaling transformation may increase the size of the model of the external support as compared to the model of the prospective pulmonary autograft by a scaling factor. The scaling factor may be between 1 and 1.25, typically between 1.05 and 1.15. This allows the (highly elastic) pulmonary autograft to assume an optimum size in the aortic position, as determined by the surgeons implanting the pulmonary autograft.

The model of the support (e.g. in the form of a CAD model) can then be used to manufacture a former from which the support for a pulmonary autograft can be made. A former is a physical model of a pulmonary autograft. Depending on the manufacturing method employed, the former can be made as follows: the CAD model file can be transferred to an appropriate Rapid Prototyping machine, e.g. selective laser sintering (SLS) to produce a physical former in a polymer, e.g. nylon. Other manufacturing techniques can be used, for example stereo lithography (SLA), CNC machining etc. Of these methods, SLS can be advantageous because the polymer(s) generally used are able to withstand being heated during a sterilisation process (where they might be heated to 134 °C for 26 minutes) or during a heat setting process.

The former produced by the above method may then be used in one of a number of manufacturing processes to make the finished support. In one method of manufacturing the finished support, a blank settable support is drawn over the former so that it is deformed to adopt a shape that morphometrically corresponds to the shape of the former.

In a second aspect, the present invention provides a method of manufacturing a support for a pulmonary autograft comprising:
(i) the method of modelling a support of the first aspect of the invention;
(ii) constructing a former from the model of support;
(iii) applying a blank support to the former.

Optionally, the method may comprise setting the shape of the support to match that of the former.

In one embodiment, the method further comprises setting the blank support to form a support which has a morphological relationship with the prospective pulmonary autograft.

In some embodiments, the step of constructing a former further comprises stretching the blank support over regions of the former where a first more supportive area is required. In some embodiments, step (iii) of the method of manufacturing a support for a pulmonary autograft according to the invention further comprises setting the stretched areas of the blank support and shrink fitting other areas of the blank support.

In a third aspect of the invention, the present invention provides a support adapted for location exteriorly of a pulmonary autograft, the support being locatable around the pulmonary autograft when placed in the aortic position and shaped to be in morphological relationship with the pulmonary autograft, the support being manufactured in accordance with the second aspect of the invention.

In one embodiment the support is in a single piece mesh which may be formed into a tube. The support may also have two longitudinal edges.

In a fourth aspect, there is further provided a method of supporting a pulmonary autograft comprising:
(i) applying a support in accordance with the third aspect of the invention to a pulmonary autograft of a patient.

In one embodiment, the support has two longitudinal edges; and the method comprises connecting the longitudinal edges such that the support forms a tube surrounding the pulmonary autograft.

There now follows, by way of example only, description of embodiments of the invention, described with reference to the accompanying drawings, in which:
**Figure 1** shows a schematic diagram depicting apparatus for use in an embodiment of the present invention;
**Figure 2** shows a flow chart setting out the steps carried out in the embodiment of Figure 1; and
**Figure 3** shows a cross section through the human heart.

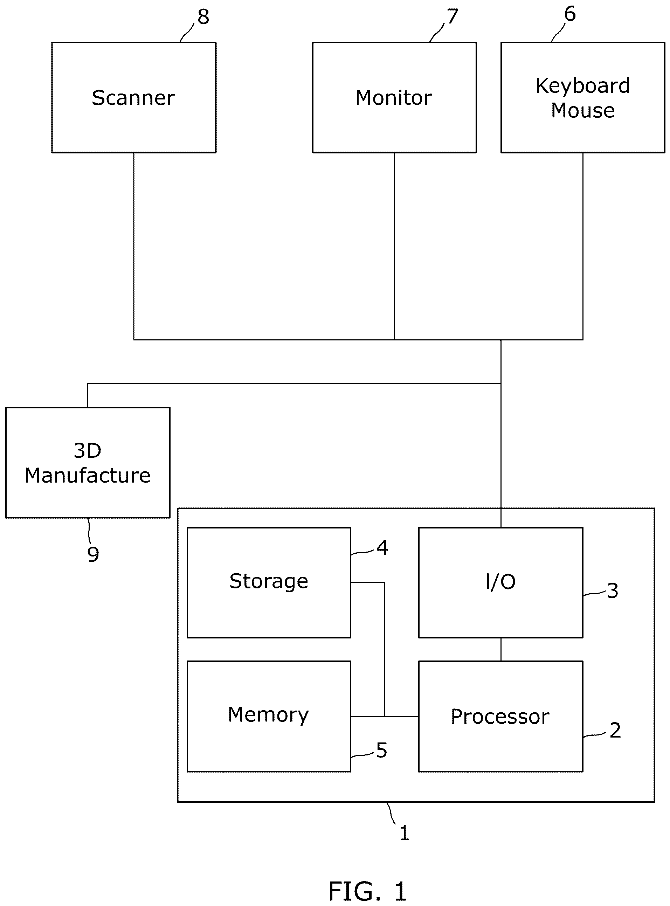

The apparatus for use in an embodiment of the invention is shown in Figure 1 of the accompanying drawings. In this embodiment, a processing computer 1 receives scans of a patient from a scanner 8; typically, this will be a standard medical computed tomography (CT) scanner, processes the scans and then can control a three-dimensional manufacturing device 9.

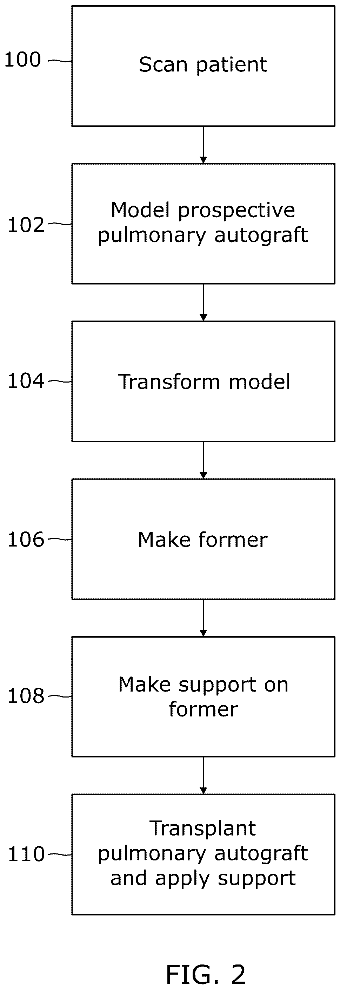

The processing computer 1 comprises a processor 2 coupled to storage 4 (e.g. a hard disk) and memory 5 (e.g. random-access memory - RAM). Input/output circuits 3 (such as network cards or other interfaces) connect the computer 1 to its control devices - a monitor 7 and keyboard and mouse 6 as well as to the scanner 8 and the 3D manufacturing device 9. The computer 1 may run any convenient operating system, such as Windows (registered trade mark; RTM), Apple MacOS (RTM) or Linux (RTM). It will be provided with suitable software to process the scans from scanner 8 and to control the 3D manufacturing device 9 as is set out below with reference to the flow chart in Figure 2 of the accompanying drawings. Whilst the discussion below is set out on the basis that the processing is carried out on a single computer, there is no reason why different computers could be involved with the tasks being distributed in any convenient manner.

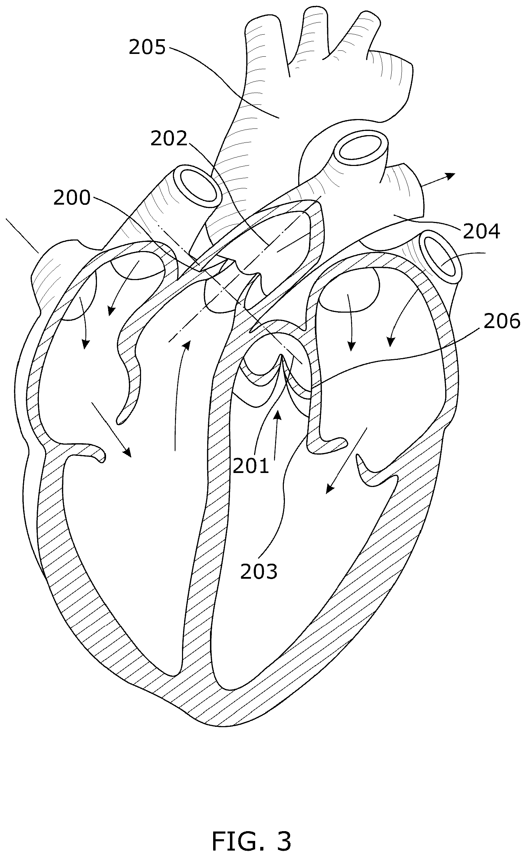

In step 100, the patient is first scanned using the scanner 8. Scan images including (references referring to Figure 3 of the accompanying drawings) of the pulmonary valve (PV) 200, and the surrounding area are taken, ideally as a single scan to avoid registration errors. The scan is taken at between 10% and 25% R-R (that is between 10% and 25% of the time from one R peak in an electrocardiogram of the patient to the next R peak), when the heart is in systole. The results of the scan will be a series of two-dimensional slices through the patient's body, which when stacked together form a three-dimensional scan of the patient (or at least their heart).

The computer 1 then transforms the three-dimension scans based around the scanner axes into images relative to the pulmonary valve 200. As such, there will be at least one image in a transaxial plane 201 in the plane of the pulmonary valve and one image in a sagittal plane 202 perpendicular thereto.

From these images, at step 102 the computer 1 can determine a number of parameters to form a model of the pulmonary valve (PV) 200, which is to form a prospective pulmonary autograft. These parameters include:
- PV 200 diameter
- Left ventricular outflow tract (LVOT) 203 diameter
- Diameter of Ascending Aorta 205
- PV axial height (that is, the distance along plane 202 from a plane of the Pulmonary ventricular junction (PVJ) to a plane of the pulmonary sinotubular junction
- PV sinotubular junction diameter
- Diameters of each of the three lobes of the PV
- Relative angles of the lobes of the PV about the centre of the lumen
- Distance of the centre of the circles defined by each the PV lobes from the centre of the lumen
- Circumference of the PV around the lobes
- Upstand pipe height - that is, how much of the PV along the pulmonary artery 204 is to be transplanted
- Upstand pipe taper - that is, how much the support must taper to transition from the PV to the aorta 205.

Where diameters are measured, they are on the basis of an approximation to a circle, be it any polygonal approximations to a circle or an equivalent diameter, i.e. the trilobal cross-section of the Sinuses of Valsalva or the essentially circular cross section of the ascending pulmonary artery.

At step 104, in order to generate a model for an external support for the pulmonary autograft, the computer 1 takes the model of the prospective pulmonary autograft and applies transformations.

The first transformation is a shape transformation which equalises the diameters of PV sinus lobes. Typically, the two smaller lobe diameters may be increased to that of the largest lobe, although alternatively all three lobes could be adjusted to the average (typically mean or median) lobe diameter. This allows a better fit of the support on the prospective pulmonary autograft, bearing in mind that the prospective pulmonary autograft once transplanted will no longer have the adjacent aorta pressing against it, and will be facing increased blood pressure compared to when it is in its original location.

As such, the shape transformation translates the centres of the lobes from forming an isosceles triangle to an equilateral triangle. The shape transformation may preserve the perimeter of the pulmonary valve.

The next transformation is a scaling transformation. Whilst typically aortic CAD models have previously been scaled on diameter only to 100%, 95% or 90% or smaller of the size of the measured vessel depending on the degree of dilation and how much reduction in diameter the surgeon considers appropriate, in the present method the scaling transformation increases the size of the model to typically between 105% to 115% of the original model. This allows the (highly elastic) pulmonary autograft to assume an optimum size in the aortic position, as determined by the surgeons implanting the pulmonary autograft.

At step 106, the model of the support (e.g. in the form of a CAD model) can then be used to manufacture a former from which the support for a pulmonary autograft can be made. The former is a physical model of a pulmonary autograft. Depending on the manufacturing method employed, the former can be made as follows: the CAD model file can be transferred to the 3D manufacturing device 9, which is an appropriate Rapid Prototyping machine, e.g. selective laser sintering (SLS). The 3D manufacturing device produces a physical former in a polymer, e.g. nylon. Other manufacturing techniques can be used, for example stereo lithography (SLA), CNC machining etc. Of these methods, SLS can be advantageous because the polymer(s) generally used are able to withstand being heated during a sterilisation process (where they might be heated to 134 °C for 26 minutes) or during a heat setting process.

In step 108, the former can then be used in one of a number of manufacturing processes to make the finished support. In this embodiment, a blank settable support is drawn over the former so that it is deformed to adopt a shape that morphometrically corresponds to the shape of the former. The support is then subjected to a setting treatment such that it retains the morphometric shape of the former, for example it may be heat set.

It is possible to stretch the blank support over regions of the former where a first more supportive area is required. The support is typically a single piece mesh which can be formed into a tube, but pre-implantation has two longitudinal edges.

In step 110, the PV 200 is transplanted to replace the damaged aortic valve 206. The support is wrapped around the transplanted pulmonary autograft and the longitudinal edges are sealed together (e.g. by stitching).

## Claims

1. A method of modelling an external support for a prospective pulmonary autograft attached to patient's heart using an imaging scanner (8), the method comprising:
(i) obtaining (100), using the scanner (8), at least one scan of the vasculature of the heart;
(ii) processing (102) the at least one scan using a processor (2) to construct a model of the prospective pulmonary autograft; and
(iii) processing (104) the model of the prospective pulmonary autograft to generate a model of the external support;
**characterised in that** the at least one scan of the vasculature of the heart is obtained during the systole of the heart.

2. The method of claim 1, in which obtaining, using the scanner (8), the at least one scan of the heart vasculature during systole of the heart comprises obtaining the at least one scan including at least one of the pulmonary valve (PV), the pulmonary trunk (PT), the aortic root and ascending aorta, the aortic valve (AV), the left ventricular outflow tract (LVOT) and the right ventricular outflow tract (RVOT).

3. The method of claim 1 or claim 2 comprising generating at least one aligned image in a plane aligned with the prospective pulmonary autograft, preferably, in which each aligned image is aligned with the pulmonary valve or the pulmonary trunk.

4. The method of claim 3, in which the aligned images include a trans-axial aligned image parallel to a plane of the pulmonary valve or perpendicular to the pulmonary trunk, and/or a sagittal aligned image perpendicular to the plane of the pulmonary valve or parallel to the pulmonary trunk.

5. The method of any preceding claim, in which the step of obtaining the at least one scan of the vasculature of the heart during systole of the heart (100) comprises obtaining the at least one scan of the vasculature of the heart in a temporal window during systole in the cardiac cycle of the patient's heart, preferably between 10% R-R and 25% R-R.

6. The method of any preceding claim in which the step of processing the at least one scan using the processor to construct the model of the prospective pulmonary autograft (102) comprises measuring one or more parameters, or at least four parameters, from the at least one scan obtained of the vasculature of the heart during systole of the heart.

7. The method of claim 6, in which the parameters include at least one of:
a) a base diameter of the left ventricular outflow tract LVOT, preferably measured at the aorto-ventricular junction
b) a diameter of each lobe of the sinuses of the prospective pulmonary autograft, relative rotational spacings of the lobes and a distance of the circular centre of the lobes from the centreline of the lumen of the prospective pulmonary autograft
c) a perimeter of the pulmonary valve
d) a diameter of the pulmonary sinotubular junction (STJ)
e) an aortic diameter
f) a height of the pulmonary valve from a plane of the pulmonary ventricular junction PVJ to a plane of the pulmonary sinotubular junction.

8. The method of any preceding claim, in which, in order to generate the model of the external support, the method comprises taking the model of the prospective pulmonary autograft and applying at least one transformation.

9. The method of claim 8 as it depends from claim 7, option b, in which the at least one transformation comprises a shape transformation comprising equalising the diameters of the lobes, preferably by translating the centres of the lobes from forming an isosceles triangle to an equilateral triangle.

10. The method of claim 9, in which the shape transformation preserves the perimeter of the pulmonary valve.

11. The method of any of claims 8 to 10, in which the at least one transformation comprises a scaling transformation, preferably, in which the scaling transformation increases the size of the model of the external support as compared to the model of the prospective pulmonary autograft, optionally by a scaling factor of between 1 and 1.2, more preferably between 1.05 and 1.15.

12. The method of any preceding claim, comprising using the model of the external support to manufacture a former from which the support for the pulmonary autograft can be made.

13. The method of claim 12, comprising forming the external support using the former.

14. The method of claim 13, in which to form the external support, a blank settable support is drawn over the former.

15. A support adapted for location exteriorly of a pulmonary autograft, the support being locatable around the pulmonary autograft and shaped to be in morphological relationship with the pulmonary autograft when placed in the aortic position, the support being manufactured in accordance with claim 13 or claim 14.

## Patentansprüche

1. Verfahren zum Modellieren eines externen Trägers für ein zukünftiges, am Herzen eines Patienten befestigtes Lungenautotransplantat unter Verwendung eines Bildgebungsscanners (8), das Verfahren umfassend:
(i) Erhalten (100), unter Verwendung des Scanners (8), von mindestens einem Scan der Blutgefäße des Herzens;
(ii) Verarbeiten (102) des mindestens einen Scans unter Verwendung eines Prozessors (2), um ein Modell des zukünftigen Lungenautotransplantats zu erstellen; und
(iii) Verarbeiten (104) des Modells des zukünftigen Lungenautotransplantats, um ein Modell des externen Trägers zu erstellen, **dadurch gekennzeichnet, dass** der mindestens eine Scan der Blutgefäße des Herzens während der Systole des Herzens erhalten wird.

2. Verfahren nach Anspruch 1, bei dem das Erhalten, unter Verwendung des Scanners (8), des mindestens einen Scans der Blutgefäße des Herzens während der Systole des Herzens das Erhalten des mindestens einen Scans umfasst, einschließlich mindestens entweder der Pulmonalklappe (PK), des Truncus pulmonalis (TP), der Aortenwurzel und der Aorta ascendens, der Aortenklappe (AK), des linksventrikulären Ausflusstrakts (LVOT) und des rechtsventrikulären Ausflusstrakts (RVOT).

3. Verfahren nach Anspruch 1 oder Anspruch 2, umfassend das Erzeugen mindestens eines ausgerichteten Bildes in einer Ebene, die an dem zukünftigen Lungenautotransplantat ausgerichtet ist, vorzugsweise, auf dem jedes ausgerichtete Bild an der Pulmonalklappe oder dem Truncus pulmonalis ausgerichtet ist.

4. Verfahren nach Anspruch 3, bei dem die ausgerichteten Bilder ein transaxiales ausgerichtetes Bild parallel zu einer Ebene der Pulmonalklappe oder senkrecht zu dem Truncus pulmonalis, und/oder ein pfeilrecht ausgerichtetes Bild, senkrecht zu der Ebene der Pulmonalklappe oder parallel zu dem Truncus pulmonalis einschließen.

5. Verfahren nach einem der vorstehenden Ansprüche, bei dem der Schritt des Erhaltens des mindestens einen Scans der Blutgefäße des Herzens während der Systole des Herzens (100) das Erhalten des mindestens einen Scans der Blutgefäße des Herzens in einem Zeitfenster während der Systole in dem Herzzyklus des Patientenherzens umfasst, vorzugsweise zwischen 10% R-R und 25% R-R.

6. Verfahren nach einem der vorstehenden Ansprüche, bei dem der Schritt des Verarbeitens des mindestens einen Scans unter Verwendung des Prozessors zum Erstellen des Modells des zukünftigen Lungenautotransplantats (102) das Messen eines oder mehrerer Parameter oder von mindestens vier Parametern aus dem mindestens einen Scan umfasst, der von den Blutgefäßen des Herzens während der Systole des Herzens erhalten wurde.

7. Verfahren nach Anspruch 6, bei dem die Parameter mindestens eines aus Folgendem einschließen:
a) Einen Basisdurchmesser des linksventrikulären Ausflusstrakts LVOT, vorzugsweise gemessen an dem aortoventrikulären Übergang
b) einen Durchmesser jedes Lappens der Sinusse des zukünftigen Lungenautotransplantats, relative Rotationsabstände der Lappen und einen Abstand der Kreismitte der Lappen von der Mittellinie der Lumen des zukünftigen Lungenautotransplantats
c) einen Umfang der Pulmonalklappe
d) einen Durchmesser des sinotubulären Übergangs der Lunge (STJ)
e) einen Aortendurchmesser
f) eine Höhe der Pulmonalklappe von einer Ebene des ventrikulären Übergangs der Lunge PVJ zu einer Ebene des sinotubulären Übergangs der Lunge.

8. Verfahren nach einem der vorstehenden Ansprüche, bei dem, um das Modell des externen Trägers zu erstellen, das Verfahren das Heranziehen des Modells des zukünftigen Lungenautotransplantats und das Anwenden mindestens einer Transformation umfasst.

9. Verfahren nach Anspruch 8, in Abhängigkeit von Anspruch 7, Option b, bei dem die mindestens eine Transformation eine Formtransformation umfasst, die das Ausgleichen des Durchmessers der Lappen umfasst, vorzugsweise durch Umwandeln der Mittelpunkte der Lappen vom Bilden eines gleichschenkligen Dreiecks zu einem gleichseitigen Dreieck.

10. Verfahren nach Anspruch 9, bei dem die Formtransformation den Umfang der Pulmonalklappe bewahrt.

11. Verfahren nach einem der Ansprüche 8 bis 10, wobei die mindestens eine Transformation eine Skalierungstransformation umfasst, vorzugsweise wobei die Skalierungstransformation die Größe des Modells des externen Trägers im Vergleich zu dem Modell des zukünftigen Lungenautotransplantats steigert, optional durch einen Skalierungsfaktor zwischen 1 und 1,2, noch mehr vorzuziehen zwischen 1,05 und 1,15.

12. Verfahren nach einem der vorstehenden Ansprüche, umfassend das Verwenden des Modells des externen Trägers, um einen Former herzustellen, aus dem der Träger für das Lungenautotransplantat gefertigt werden kann.

13. Verfahren nach Anspruch 12, umfassend das Bilden des externen Trägers unter Verwendung des Formers.

14. Verfahren nach Anspruch 13, bei dem zur Bildung des externen Trägers ein leerer festlegbarer Träger über den Former gezogen wird.

15. Träger, der zur Anordnung außerhalb eines Lungenautotransplantats angepasst ist, wobei der Träger um das Lungenautotransplantat herum angeordnet und so geformt ist, dass er in morphologischer Beziehung zu dem Lungenautotransplantat steht, wenn er in der Aortenposition angeordnet ist, wobei der Träger nach Anspruch 13 oder Anspruch 14 hergestellt ist.

## Revendications

1. Procédé de modélisation d'un support externe pour une autogreffe pulmonaire prospective fixée au cœur d'un patient, à l'aide d'un tomodensitomètre d'imagerie (8), le procédé comprenant les étapes consistant à :
(i) obtenir (100), en utilisant le tomodensitomètre (8), au moins une image tomodensitométrique du système vasculaire du cœur ;
(ii) traiter (102) ladite au moins une image tomodensitométrique, en utilisant un processeur (2) pour construire un modèle de l'autogreffe pulmonaire prospective ; et
(iii) traiter (104) la modèle de l'autogreffe pulmonaire prospective pour générer un modèle du support externe ;
**caractérisé en ce que** ladite au moins une image tomodensitométrique du système vasculaire du cœur est obtenue pendant la systole du cœur.

2. Procédé selon la revendication 1, dans lequel obtenir, en utilisant le tomodensitomètre (8), ladite au moins une image tomodensitométrique du système vasculaire du cœur pendant la systole du cœur comprend obtenir ladite au moins une image tomodensitométrique incluant au moins une de la valve pulmonaire (VP), du tronc pulmonaire (TP), de la racine aortique et l'aorte ascendante, de la valve aortique (VA), de la voie d'éjection du ventricule gauche (VEVG) et de la voie d'éjection du ventricule droit (VEVD).

3. Procédé selon la revendication 1 ou la revendication 2, comprenant générer au moins une image alignée dans un plan aligné avec l'autogreffe pulmonaire prospective, de préférence, dans lequel chaque image alignée est alignée avec la valve pulmonaire ou le tronc pulmonaire.

4. Procédé selon la revendication 3, dans lequel les images alignées incluent une image alignée transaxiale parallèle à un plan de la valve pulmonaire ou perpendiculaire au tronc pulmonaire, et/ou une image alignée sagittale perpendiculaire au plan de la valve pulmonaire ou parallèle au tronc pulmonaire.

5. Procédé selon l'une quelconque des revendications précédentes, dans lequel l'étape consistant à obtenir (100) ladite au moins une image tomodensitométrique du système vasculaire du cœur pendant la systole du cœur comprend obtenir ladite au moins une image tomodensitométrique du système vasculaire du cœur dans une fenêtre temporelle pendant la systole dans le cycle cardiaque du cœur d'un patient, de préférence entre 10 % de R-R et 25 % de R-R.

6. Procédé selon l'une quelconque des revendications précédentes, dans lequel l'étape consistant à traiter (102) ladite au moins une image tomodensitométrique, en utilisant le processeur, pour construire le modèle de l'autogreffe pulmonaire prospective comprend mesurer un ou plusieurs paramètres, ou au moins quatre paramètres, d'après ladite au moins une image tomodensitométrique obtenue du système vasculaire du cœur pendant la systole du coeur.

7. Procédé selon la revendication 6, dans lequel les paramètres incluent au moins un(e) parmi :
a) un diamètre de base de la voie d'éjection du ventricule gauche (VEVG), mesuré de préférence au niveau de la jonction aorto-ventriculaire
b) un diamètre de chaque lobe des sinus de l'autogreffe pulmonaire prospective, les espacements rotationnels relatifs des lobes et une distance du centre du cercle des lobes à partir de la ligne centrale de la lumière de l'autogreffe pulmonaire prospective
c) un périmètre de la valve pulmonaire
d) un diamètre de la jonction sinotubulaire (JST) pulmonaire
e) un diamètre de l'aorte
f) une hauteur de la valve pulmonaire à partir d'un plan de la jonction ventriculaire pulmonaire JVP jusqu'à un plan de la jonction sinotubulaire pulmonaire.

8. Procédé selon l'une quelconque des revendications précédentes, dans lequel, afin de générer le modèle du support externe, le procédé comprend prendre le modèle de l'autogreffe pulmonaire prospective et appliquer au moins une transformation.

9. Procédé selon la revendication 8 telle que dépendante de la revendication 7, option b, dans lequel ladite au moins une transformation comprend une transformation de forme comprenant égaliser les diamètres des lobes, de préférence en opérant une translation des centres des lobes conduisant de la formation d'un triangle isocèle à celle d'un triangle équilatéral.

10. Procédé selon la revendication 9, dans lequel la transformation de forme conserve le périmètre de la valve pulmonaire.

11. Procédé selon l'une quelconque des revendications 8 à 10, dans lequel ladite au moins une transformation comprend un changement d'échelle, de préférence dans lequel le changement d'échelle augmente la taille du modèle du support externe en comparaison du modèle de l'autogreffe pulmonaire prospective, en option par un facteur d'échelle compris entre 1 et 1,2, plus préférablement entre 1,05 et 1,15.

12. Procédé selon l'une quelconque des revendications précédentes, comprenant utiliser le modèle du support externe pour fabriquer un gabarit à partir duquel peut être produit le support pour l'autogreffe pulmonaire.

13. Procédé selon la revendication 12, comprenant former le support externe en utilisant le gabarit.

14. Procédé selon la revendication 13, dans lequel pour former le support externe un support vierge ajustable est amené sur le gabarit.

15. Support adapté au placement par l'extérieur d'une autogreffe pulmonaire, le support pouvant être placé autour de l'autogreffe pulmonaire et façonné pour être en relation morphologique avec l'autogreffe pulmonaire lorsqu'elle est placée dans la position aortique, le support étant fabriqué conformément à la revendication 13 ou la revendication 14.
